(19) Europäisches Patentamt / European Patent Office / Office européen des brevets

(11) **EP 3 145 334 B1**

(12) **EUROPEAN PATENT SPECIFICATION**

(45) Date of publication and mention
of the grant of the patent:
**13.11.2019  Bulletin 2019/46**

(21) Application number: **14729065.4**

(22) Date of filing: **19.05.2014**

(51) Int Cl.:
**A23L 33/17** (2016.01)    **A23L 33/00** (2016.01)

(86) International application number:
**PCT/NL2014/050315**

(87) International publication number:
**WO 2015/178762 (26.11.2015 Gazette 2015/47)**

(54) **ENTERAL FORMULATIONS FOR PRETERM INFANTS COMPRISING OPTIMISED PHENYLALANINE INTAKE LEVELS**

FRÜHGEBORENE ENTERAL-FORMULIERUNGEN DIE EINE OPTIMIERTE PHENYLALANIN-EINNAHMEMENGE ERLAUBEN

FORMULATIONS ENTERALES POUR PREMATURES PERMETTANT D'INGERER UNE QUANTITE DE PHENYLALANINE OPTIMISEE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.03.2017  Bulletin 2017/13**

(73) Proprietor: **N.V. Nutricia**
**2712 HM  Zoetermeer (NL)**

(72) Inventors:
- **VAN DER BEEK, Eline Marleen**
  **3584 CT Utrecht (NL)**
- **ABRAHAMSE-BERKEVELD, Marieke**
  **3584 CT Utrecht (NL)**
- **VAN GOUDOEVER, Johannes Bernard**
  **3584 CT Utrecht (NL)**
- **TELLER, Inga Christiane**
  **3584 CT Utrecht (NL)**

(74) Representative: **Nederlandsch Octrooibureau**
**P.O. Box 29720**
**2502 LS The Hague (NL)**

(56) References cited:
**EP-A1- 1 932 437        EP-A2- 0 418 593**

WO-A1-2007/121807    WO-A1-2010/112430
WO-A1-2011/119023

- **BALLHAUSEN ET AL.: "Born at 27 weeks of gestation with classical PKU: challenges of dietetic management in a very preterm infant", PEDIATRIC REPORTS, vol. 3:e26, 22 September 2011 (2011-09-22) , pages 103-107, XP002735149,**
- **DATABASE CAPLUS [Online] CHEMICAL ABSTRACTS SERVICE, COLUMBUS, OHIO, US; 12 May 1984 (1984-05-12), Guettler et al.: "Serum tyrosine within the first hour after an oral lod of phenylalanine", XP002737745, retrieved from stn Database accession no. 1978:488340**
- **JACOMINE E HOGEWIND-SCHOONENBOOM ET AL: "Phenylalanine requirements of enterally fed term and preterm neonates", THE AMERICAN JOURNAL OF CLINICAL NUTRITION, vol. 101, no. 6, 29 April 2015 (2015-04-29), pages 1155-1162, XP55462005, US ISSN: 0002-9165, DOI: 10.3945/ajcn.114.089664**
- **C Agostoni ET AL: "Enteral Nutrient Supply for Preterm Infants: Commentary From the European Society of Paediatric Gastroenterology, Hepatology and Nutrition Committee on Nutrition", Journal of Pediatric Gastroenterology and Nutrition, vol. 50, no. 1, 1 January 2010 (2010-01-01), pages 85-91, XP55099146, ISSN: 0277-2116, DOI: 10.1097/MPG.0b013e3181adaee0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The present invention relates to the determination of optimal intake levels of phenylalanine for preterm infants, and to enteral formulations (*e.g.*, preterm formulas and human milk fortifiers) that achieve these optimal intake levels. The present invention also relates to the administration of these enteral formulations to preterm infants, particularly in order to promote, assist or achieve balanced growth and/or development of the preterm infants.

BACKGROUND

Protein accretion is a crucial part of growth, especially during foetal life and early infancy, which explains at least in part the higher needs for protein of preterm infants.

The protein composition in preterm human milk (*i.e.,* human milk from a mother who has delivered a preterm infant) differs from term human milk (Zhang 2013) to compensate at least in part for these higher needs. However, although this transitory compensation mechanism takes place in preterm milk composition, the high energy, protein, mineral, and vitamin requirements of a preterm infant may not always be met in the preterm human milk. But as human milk is the preferred feeding for preterm infants, required for healthy gut development, prevention of infection and reducing the risk of potentially fatal diseases such as necrotising enterocolitis, fortification of preterm milk has been recommended by ESPGHAN (Agostoni, 2010) to provide all the benefits of human milk and also meet the additional nutritional support required by these vulnerable infants. Only when maternal or donor milk is not available for fortification, a preterm formula particularly composed to meet requirements of preterm infants can be considered (Agostoni, 2010). Preterm formulas specifically designed for preterm infants thus contain higher levels of protein compared to infant formulas for term infants so that the increased protein requirements of these fragile infants can be met.

Ballhausen et al., Pediatric Reports 2011 vol.3 p.103-107 relates to challenges of dietetic management in a very preterm infant.

Although the protein needs of preterm infants has been intensively studied, thus leading to many publications and ESPGHAN guidelines on the protein intake levels in preterm formulas, there has not been so much attention paid to the quality of the protein needed by preterm infants. The protein component of enteral formulations for preterm infants (*e.g.*, preterm formulas, human milk fortifiers, other protein supplements), like all infant formulas, is made from non-human milk sources. Typically bovine milk is used, but other animal milk sources including caprine (goat's) milk may be used, or non-animal protein sources such as vegetable (e.g. soy) protein may be used. None of these sources match exactly the protein quality of human milk.

Protein quality is largely determined by the amino acid composition of protein, as amino acids are the building blocks of protein. One of the amino acids, phenylalanine, plays a number of important metabolic roles. This essential aromatic amino acid serves as a precursor via phenylalanine hydroxylation to tyrosine, the monoamine signalling molecules dopamine, norepinephrine, and epinephrine, and the skin pigment melanin.

Symptoms of phenylalanine deficiency include confusion, lack of energy, depression, decreased alertness, memory problems, and lack of appetite. On the other hand, phenylalanine in large quantities can interfere with the production of serotonin, a neurotransmitter derived from tryptophan and involved in gut motility regulation that has been linked to feelings of well-being and happiness in adults. Further, the rare metabolic disorder phenylketonuria (PKU) occurs when a specific enzyme to use phenylalanine is missing. This causes a build-up of high phenylalanine concentrations that if not treated before 3 weeks of age can cause severe, irreversible mental and intellectual disability.

It is therefore important to find the right balance of phenylalanine in a young infant's diet, especially in the preterm infant where additional risks for healthy and normal growth and/or development exist.

SUMMARY

The present inventors researched phenylalanine uptake and protein synthesis in preterm infants and discovered that a preterm infant's requirements of phenylalanine do not appear to be in line with existing preterm formulations, including preterm formulas and human milk fortifiers.

More specifically, the inventors have found that a preterm infant's requirements of phenylalanine are lower than the amounts currently recommended for and applied in preterm formulas and human milk fortifiers. The inventors established the phenylalanine requirement of preterm infants to be 80 mg/kg/day, which is 80-96 mg/kg per day with a 20% variation permitted (e.g. to compensate for product-allowed variation). The requirement of 80 mg/kg per day was subsequently determined to be about 2.0-2.29 g / 100 g (protein) and 64.00-82.29 mg / 100 kcal. In the context of human milk fortifiers, these levels correspond to 0-11.52 mg / 100 kcal or 0-9.72 mg / g (protein). These findings suggest that the phenylalanine levels required by preterm infants are considerably (and unexpectedly) lower than those found in existing preterm formulations such as preterm formulas and human milk fortifiers. For instance, in cow's milk-based preterm formulas, phenylalanine levels have been reported to vary between 3.54-4.53 g (phenylalanine) / 100 g protein (Picaud, 2001; Moro, 1999; Darling, 1999; Szajewska, 2001) or 91.64-118.25 mg (phenylalanine) / 100 kcal (Picaud, 2001; Moro, 1999; Darling, 1999; Szajewska, 2001).

Thus based on the inventors' findings, it is proposed that preterm formulations, especially preterm formulas and human milk fortifiers, can be produced with reduced phenylalanine concentrations.

Furthermore, based on the competition between amino acids and need for bioavailability of amino acids in infants (especially in preterm infants), it is proposed that such preterm formulas can be produced with an optimised amino acid profile. For example, a preterm formula with a lowered phenylalanine concentration will require a relative increase of other amino acids in the formula in order to maintain a desired total protein intake level for preterm infants between 3.5-4.0 g protein / kg / day (Agostoni, 2010). It is proposed that such preterm formulas can better meet the needs of preterm infants, thus promoting better growth and/or development in this vulnerable target group.

Thus, according to one aspect of the present invention, we provide an enteral preterm infant formula comprising a proteinaceous composition comprising an optimised phenylalanine concentration, wherein the formula:

a) comprises 2.0-2.29 phenylalanine per 100 g protein;
b) comprises 64.00-82.29 mg phenylalanine per 100 kcal; and/or
c) provides, or is formulated to provide phenylalanine in an amount of 80-96 mg per kg body weight per day.

The proteinaceous composition may comprise intact proteins, hydrolysed proteins, protein fractions, free amino acids and/or a combination thereof. Based on the inventors' findings as outlined herein, in some embodiments the proteinaceous composition comprises 2.0-2.29 g phenylalanine per 100 g protein. Suitably, the proteinaceous composition comprises 2.0-2.25 g, more suitably 2.05-2.20 g, more suitably 2.05-2.15 g, most suitably 2.1-2.15 g phenylalanine per 100 g protein.

There is provided, according to one aspect of the present invention an enteral preterm infant formula comprising the proteinaceous composition as defined above. In addition or alternatively, there is provided a preterm formula comprising 64.00-82.29 mg phenylalanine per 100 kcal and/or providing, or being formulated to provide phenylalanine in a concentration of 80-96 mg per kg body weight per day (mg/kg body weight/day). Suitably, the formula comprises 64.00-82.29 mg phenylalanine per 100 kcal and provides, or is formulated to provide, phenylalanine in an amount of 80-96 mg per kg body weight per day.

In one aspect of the invention, there is provided an enteral preterm infant formula comprising a proteinaceous composition, wherein the formula:

a) comprises 2.0-2.29 phenylalanine per 100 g protein;
b) comprises 64.00-82.29 mg phenylalanine per 100 kcal; and/or
c) provides, or is formulated to provide phenylalanine in an amount of 80-96 mg per kg body weight per day.

According to another aspect of the present invention, there is provided a preterm formula as defined herein for use in promoting, assisting or achieving balanced growth or development in a preterm infant.

As another aspect of the present invention, there is provided a preterm formula as defined herein for use in preventing or reducing the risk of unbalanced growth or development in a preterm infant. The formula is a preterm formula comprising the proteinaceous composition as described in the context of the invention as described herein.

Not pertaining to the invention: the term "growth or development" may refer to growth and development of the brain of a preterm infant and/or the cognitive function of the infant as discussed further below, and/or to growth and development of the body of a preterm infant and/or the infant's body composition, also as discussed further below.

The proteinaceous compositions and preterm formulas of the various aspects of the invention are discussed in more detail further below.

## BRIEF DESCRIPTION OF THE FIGURES

Figure 1 shows an example of a graph plotted by the IAAO method for a test amino acid as described in the introduction for Example 1.

Figure 2 shows the rates of release of $^{13}CO_2$ in the breath of 16 preterm infants ($F^{13}CO_2$) at 24 different phenylalanine intakes as described in Example 1. Using a biphasic linear regression crossover model, the mean breakpoint was estimated to be 80 mg·kg$^{-1}$·d$^{-1}$ ($P < 0.01$, r$^2$ = 0.54).

The embodiments will be outlined here below.

## DETAILED DESCRIPTION OF THE INVENTION

*Proteinaceous Compositions*

The present invention is based on the inventors' discovery of the requirements of preterm infants for phenylalanine by actual measurements of requirement intake levels in formula-fed preterm infants, and the subsequent determination of those intake levels in the context of product (*e.g.*, concentrations in preterm formulations such as preterm formulas and human milk fortifiers).

Interestingly, it is noted that the intake requirement of phenylalanine (or phenylalanine concentrations) deviates from the concentrations used in commercially available preterm formulas. For instance, in cow's milk-based preterm formulas, phenylalanine concentrations have been reported to vary between 3.54-4.53 g (phenylalanine) / 100 g protein (Picaud, 2001; Moro, 1999; Darling, 1999; Szajewska, 2001) or 91.64-118.25 mg (phenylalanine) / 100 kcal (Picaud, 2001; Moro, 1999; Darling, 1999; Szajewska, 2001). By contrast, the inventors surprisingly discovered that the intake level of phenylalanine required for protein synthesis (and therefore for growth and development) in preterm infants are met at a lower concentration. These results also suggest that preterm formulas based on this discovery that maintain the same protein levels as existing preterm formulas, must have an altered ratio between phenylalanine and other amino acids, especially large neutral amino acids (valine, leucine, isoleucine, tryptophan, tyrosine and methionine). These insights could mean that by reducing phenylalanine concentrations relative to the other large neutral amino acids, the large neutral amino acids involved in protein accretion will have a better bioavailability due to less transporter competition. As a result, the lower ratio of phenylalanine to large neutral amino acids may induce a higher growth in lean body mass (muscle accretion) as well as brain development by increased cerebral protein synthesis.

Furthermore, too high concentrations of phenylalanine might interfere with the development of balanced growth and/or body composition, including lean body mass, by influencing the uptake of large neutral amino acids. Thus, using the new phenylalanine concentrations and optionally a lower ratio of phenylalanine to large neutral amino acids in proteinaceous compositions and preterm formulations (*e.g.*, preterm formulas or human milk fortifiers) proposed herein, may allow for compositions that promote balanced growth and/or development, as further described herein.

The invention thus pertains to an enteral preterm infant formula comprising a proteinaceous composition comprising 2.0-2.29 g phenylalanine per 100 g protein, suitably 2.0-2.25 g phenylalanine per 100 g protein, more suitably 2.05-2.20 g phenylalanine per 100 g protein, even more suitably 2.05-2.15 g phenylalanine per 100 g protein, most suitably 2.10-2.15 g phenylalanine per 100 g protein.

The proteinaceous composition may comprise intact proteins, hydrolysed proteins, protein fractions, free amino acids and/or a combination thereof. Where hydrolysed proteins are used, they may comprise partially and/or extensively hydrolysed proteins.

The proteinaceous composition of any aspect of the present invention may comprise a ratio of essential amino acids to non-essential amino acids of 40-60 : 40-60, suitably 45-55 : 45-55, even more suitably 48-52 : 48-52.

The proteinaceous composition is part of an enteral preterm infant formula, and is intended for a preterm infant. Therefore, throughout this specification, the term "preterm infant" (or "preterm") means an infant born before completion of 37 weeks of gestation. Suitably, the term "preterm infant" refers to an infant weighing >1000 g at birth, most suitably the term "preterm infant" refers to an infant weighing 1000-1800 g at birth.

*Preterm Formulations*

The term "preterm formulation" as used herein is intended to encompass all formulations (*e.g.* formulas and fortifiers) intended for a preterm infant. Exemplary preterm formulations include preterm formulas and also human milk fortifiers as herein defined (*e.g.,* those which are intended to be admixed with human milk before administration to a preterm infant), and also a composition comprising human milk and a human milk fortifier.

Accordingly, an aspect of the present invention provides an enteral preterm infant formula comprising a proteinaceous composition, wherein the formula:

    a) comprises 2.0-2.29 g phenylalanine per 100 g protein;
    b) comprises 64.00-82.29 mg phenylalanine per 100 kcal; and/or
    c) provides, or is formulated to provide phenylalanine concentrations of 80-96 mg / kg /day.

In an exemplary embodiment, the formula complies with at least (a). Suitably, the formula complies with at least both (a) and (b), with at least both (a) and (c), or with at least both (a), (b) and (c). In another embodiment, the formula complies with at least (b), suitably complies with at least both (b) and (c).

Suitably, the formula comprises 2.0-2.25 g phenylalanine per 100 g protein, more suitably 2.05-2.20 g, even more suitably 2.05-2.15 g, most suitably 2.10-2.15 g phenylalanine per 100 g protein.

Suitably, the formula comprises 65-81 mg phenylalanine per 100 kcal, more suitably 67-79 mg phenylalanine per 100 kcal, even more suitably 69-77 mg phenylalanine per 100 kcal, most suitably 71-75 mg phenylalanine per 100 kcal.

Suitably, the formula provides, or is formulated to provide phenylalanine in an amount of 82-95 mg / kg body weight / day, more suitably in an amount of 83-92 mg / kg body weight / day, even more suitably in an amount of 84-90 mg / kg body weight / day, most suitably in an amount of 85-88 mg / kg body weight / day.

The proteinaceous composition of any aspect of the present invention may comprise a ratio of essential amino acids to non-essential amino acids of 40-60 : 40-60, suitably 45-55 : 45-55, even more suitably 48-52 : 48-52.

The preterm formula of any aspect of the present invention may be intended as a complete nutrition for infants. Suitably, the preterm formula of any aspect of the present invention is intended for a preterm infant. Suitably, the preterm formula of any aspect of the present invention is intended for a human preterm infant.

The proteinaceous composition or preterm formula of the invention is typically intended for preterm infants from birth to hospital discharge and/or upon discretion of the health care professional.

The preterm formula of the present invention is an enteral composition, *i.e.,* any composition that is enterally administered, such as orally. As used in this document, the term "enteral" is intended to refer to the delivery directly into the gastrointestinal tract of a subject (*e.g.*, orally or via a tube, catheter or stoma), as opposed to a direct delivery into the blood stream (parenteral feeding).

The proteinaceous composition or preterm formula of the present invention may be formulated for administration in a liquid form. In some embodiments, the preterm formula may comprise a powder suitable for making a liquid composition after reconstitution with an aqueous solution. The preterm formula may be made up as a packaged powder composition wherein the package is provided with instructions to admix the powder with a suitable amount of aqueous solution, thereby resulting in a liquid composition. In some other embodiments, the preterm formula may comprise a ready-to-use liquid food (*e.g.,* is in a ready-to-feed liquid form). A packed ready-to-use liquid food may involve fewer steps for preparation than a powder to be reconstituted and hence may involve a reduced chance on contamination by harmful micro-organisms.

## (i) Preterm Formulas

Preterm formulas are employed with preterm infants when maternal milk or donor milk (optionally with fortification) is not available. The present invention provides preterm formulas as herein defined.

The protein intake recommendation of preterm infants with a birth weight of less than 1000 g is 4.0-4.5 g·kg$^{-1}$d$^{-1}$. Accordingly, the preterm formula of the present invention may comprise a protein concentration corresponding to a protein intake of 4.0-4.5 g·kg$^{-1}$d$^{-1}$, suitably 4.1-4.5 g·kg$^{-1}$d$^{-1}$, more suitably 4.2-4.4 g·kg$^{-1}$d$^{-1}$.

The preterm formula of the present invention may comprise 3.2-4.1 g protein per 100 kcal, suitably 3.6-4.1 g protein per 100 kcal, more suitably 3.7-4.1 g protein per 100 kcal. Suitably, such preterm formula is intended for a preterm infant weighing < 1000 g at birth.

The protein intake recommendation of preterm infants with a birth weight between 1000-1800 g is 3.5-4.0 g·kg$^{-1}$d$^{-1}$. Accordingly, the preterm formula of the present invention may comprise a protein concentration corresponding to a protein intake of 3.5-4.0 g·kg$^{-1}$d$^{-1}$, suitably 3.6-4.0 g·kg$^{-1}$d$^{-1}$, more suitably 3.7-3.9 g·kg$^{-1}$d$^{-1}$.

In other embodiments, the preterm formula of the present invention may comprise 3.2-3.6 g protein per 100 kcal, suitably 3.3-3.6 g protein per 100 kcal, more suitably 3.4-3.5 g protein per 100 kcal. Suitably, such preterm formula is intended for a preterm infant weighing 1000-1800 g at birth.

The preterm formula of the present invention may further comprise a lipid, a carbohydrate, a vitamin and/or a mineral. In some embodiments, the preterm formula may comprise between 5 and 50 en% lipid, between 5 and 50 en% protein, between 15 and 90 en% carbohydrate. Suitably, the preterm formula may comprise between 35 and 50 en% lipid, between 7.5 and 18 en% protein and between 35 and 80 en% carbohydrate (en% is short for energy percentage and represents the relative amount each constituent contributes to the total caloric value of the preparation). The preterm formula may provide a carbohydrate intake of 10-14 g / 100 kcal, suitably 10.5-12 g/ 100 kcal.

The preterm formula may further comprise a non-digestible oligosaccharide. Suitably, the non-digestible oligosaccharide may be selected from the group consisting of galacto-oligosaccharides, fructo-oligosaccharides and acidic oligosaccharides. Such oligosaccharides are well known to those skilled in the art.

The preterm formula may further comprise a polyunsaturated fatty acid (PUFA). Suitably, the PUFA may be selected from the group consisting of alpha-linolenic acid (ALA), linoleic acid (LA), eicosapentaenoic acid (EPA), docosahexaenoic acid (DHA), arachidonic acid (ARA), and docosapentaenoic acid (DPA). Suitably, the PUFA may be a long chain polyunsaturated fatty acid (LCPUFA) (*e.g.*, EPA, DHA, ARA, DPA). Such PUFAs are well known to those skilled in the art.

The preterm formula may further comprise a probiotic. Suitable probiotics are well known to those skilled in the art. The probiotic may comprise a lactic acid producing bacterium. The probiotic may comprise a *Lactobacillus* species. The probiotic may comprise a *Lactobacillus rhamnosus* strain (including *L. rhamnosus* GG, also referred to as "LGG"), a *Lactobacillus salivarius* strain, a *Lactobacillus casei* strain, a *Lactobacillus paracasei* strain (including *L. paracasei* F19), a *Lactobacillus acidophilus* strain, a *Lactobacillus reuteri* strain, and/or a *Lactobacillus helveticus* strain. The probiotic may comprise a *Bifidobacterium* species. The probiotic may comprise a *Bifidobacterium longum* strain, a *Bifidobacterium infantis* strain, a *Bifidobacterium breve* strain (including *B. breve* M-16V and *B. breve* BbC50), a *Bifidobacterium animalis* strain (including *B. animalis* subsp. *lactis,* including subsp. *lactis* BB-12 and subsp. *lactis* Bi-07), and/or a *Bifidobacterium bifidum* strain. In some embodiments, the probiotic may be viable or non-viable. As used herein, the term "viable", refers to live microorganisms. The term "non-viable" or "non-viable probiotic" means non-living probiotic microorganisms, their cellular components and metabolites thereof. Such non-viable probiotics may have been heat-killed or otherwise inactivated but retain the ability to favourably influence the health of the host. The probiotics may be naturally-occurring, synthetic or developed through the genetic manipulation of organisms, whether such new source is now known or later developed. Suitably, the preterm formula does not comprise *Saccharomyces cerevisiae.*

The preterm formula may comprise a fermented or a non-fermented composition. Fermentation by micro-organisms results in a lowering of the pH. As a non-fermented composition, the preterm formula may have a pH above 5.5, such as 6.0, such as 6.5 (for example, in order to reduce damage to teeth). The pH may suitably be between 6 and 8.

The preterm formula may be formulated to provide an energy intake of 110-135 kcal / kg / day, or 72-200 kcal/100 ml, suitably 76-120 kcal/100 ml, more suitably 77-100 kcal/100 ml, even more suitably 78-90 kcal/100 ml, most suitably 79-83 kcal/100 ml.

The preterm formula may have a long shelf life. For example, it may be shelf stable at ambient temperature for at least 6 months, such as least 12 months, where it is in a liquid, ready-to-feed form or where it is in a powder form.

Suitably, the preterm formulas of the present invention do not comprise or consist of human breast milk.

*Protein Sources*

The proteinaceous compositions or preterm formulas of the present invention may comprise an intact protein, a hydrolysed protein, a protein fraction, a free amino acid and/or a combination thereof, such that the proteinaceous composition or preterm formula comprises the amino acid profile of any aspect of the present invention.

The term "an intact protein" as used herein refers to any form of intact protein, including but not limited to a protein concentrate and/or a protein isolate, as well as other forms of intact proteins.

The term "hydrolysed protein" as used herein refers to partially and/or extensively hydrolysed proteins. Suitably the proteinaceous composition of the present invention comprises a hydrolysed protein with a degree of hydrolysis of between 5% and 25%, more suitably between 7.5% and 21%, most suitably between 10% and 20%. The degree of hydrolysis is defined as the percentage of peptide bonds which have been broken by enzymatic hydrolysis, with 100% being the total potential peptide bonds present. A suitable method for preparing a protein hydrolysate is described in WO 2001/041581, the entire contents of which is incorporated herein by reference.

The proteinaceous composition or preterm formula may comprise any suitable intact protein, hydrolysed protein, protein fraction, free amino acid and/or a combination thereof, which is selected ensuring that the phenylalanine concentration of the proteinaceous composition or preterm formula as defined herein are met. For instance, as discussed elsewhere, the present inventors have demonstrated the desirability of lowering the concentrations of phenylalanine from those found in commercially available preterm formulations and/or for lowering the concentration of phenylalanine relative to that of the other large neutral amino acids.

In some embodiments, the proteinaceous composition or preterm formula comprises an intact protein, and a free amino acid. In some embodiments, the proteinaceous composition or preterm formula comprises a hydrolysed protein and/or a protein fraction, and a free amino acid. In some embodiments, the proteinaceous composition or preterm formula comprises free amino acids. In some embodiments, the proteinaceous composition or preterm formula consists essentially of or consists of free amino acids.

The proteinaceous composition or preterm formula may comprise a non-human animal protein (such as milk proteins, including caseins and whey proteins, meat proteins and egg proteins), a non-animal protein, a dairy protein, a non-cow dairy protein, a non-dairy protein, a vegetable protein, an algal protein, a hydrolysate of any of these proteins, a fraction of any of these proteins, a free amino acid, and/or a combination of any of these amino acid sources.

Suitably, the proteinaceous composition or preterm formula comprises at least 40%, 50%, 60%, 70%, 80%, 90%, or 95% (or any integer in-between) of amino acid sources comprising an intact protein, a hydrolysed protein, a protein fraction, such that the proteinaceous composition comprises less than 5%, 10%, 20%, 30%, 40%, 50%, or 60% (or any integer in-between) of a free amino acid. Most suitably, the proteinaceous composition or preterm formula comprises at least 60% of amino acid sources comprising an intact protein, a hydrolysed protein, a protein fraction, such that the proteinaceous composition comprises 40% or less of a free amino acid.

Reference throughout this document (both specification and claims) is made to weight units (milligrams or grams) of amino acids. Where expressed per weight unit of protein (*e.g.,* per 100 g protein), it should be noted that this expression refers to the relative weight of the amino acid(s) in terms of the protein weight, where the protein weight means the weight of all protein matter.

Furthermore, where reference throughout this document (both specification and claims) is made to weight units (milligrams or grams) of amino acids, it should be noted that these units are protein equivalent weights of amino acids (*i.e.,* the weight of amino acid when present in a protein). Thus, where a free amino acid is employed in the present invention, the dehydration synthesis reactions which occur when a protein is formed from free amino acids must be taken into account. Accordingly, if a free amino acid is employed in the present invention, the weight of the free amino acid that is required is 17% higher than the protein equivalent weight (as expressed herein). Such calculations and conversions are well known to those skilled in the art.

Suitable amino acid sources for the proteinaceous composition or preterm formulas may include, but are not limited to, cow's milk protein, whey (including acid whey, sweet whey, and alpha-lactalbumin enriched whey), alpha-lactalbumin, betalactoglobulin, glycomacropeptide, casein (including beta-casein), skim milk, lactoferrin, colostrum, goat's milk protein, donkey's milk protein, buffalo milk protein, fish, chicken protein, pork protein, soy protein (including soy protein isolate), pea protein (including pea protein isolate), wheat protein, rice protein, rice bran, potato protein (including potato protein isolate), another plant protein, fractions of any of these proteins, hydrolysates of any of these proteins, and free amino acids (including amino acids isolated from an amino acid source and/or amino acids that have been chemically or synthetically produced). Suitably, the proteinaceous composition and/or the preterm formula of the present invention comprise glycomacropeptide.

The amino acid profile of the proteinaceous composition or preterm formula of the present invention, as described herein, refers to one or more essential amino acids (or conditionally essential amino acids in some instances). It will be recognised by those skilled in the art that the proteinaceous composition or preterm formula will also comprise non-essential amino acids. Suitably, the proteinaceous composition or preterm formula of the present invention comprises a ratio of essential amino acids to non-essential amino acids of 40-60 : 40-60, suitably 45-55 : 45-55, even more suitably 48-52 : 48-52. This is in line with the typical ratio of essential amino acids in human milk and commercially available formulas, thus it is noted that the present invention does not contemplate a lowering of the essential amino acids relative to the non-essential amino acids.

*Balanced growth and/or development*

As described herein, the present inventors have developed optimised proteinaceous compositions and preterm formulas. The optimised phenylalanine concentrations (or ratio of phenylalanine to large neutral amino acids) in these proteinaceous compositions and preterm formulas is designed from the real preterm infants' requirements that were measured as described herein. Therefore, these concentrations meet the needs of preterm infants and thus promote balanced growth and/or development in a preterm infant, and/or prevent or reduce the risk of unbalanced growth and/or development in a preterm infant.

The term "balanced" as used in the phrase "balanced growth and/or development" is intended to refer to healthy or normal growth and/or development of a preterm infant. For example, the growth and/or development is not too low/little or too high/much ("unbalanced growth and/or development"). In preterm infants, this often means a steady increase in weight and/ or length and/or head circumference, and/or no growth faltering in any of these anthropometric parameters. Balanced growth and/or development as used herein may be with reference to published figures that define the (*e.g.,* healthy or normal) growth and/or development of a preterm infant with reference to a population (geographic, demographic, ethnic, *etc*) within which the infant belongs, or with reference to the growth and/or development an unhealthy infant or group of unhealthy infants, such as the Fenton Growth charts for preterm infants (http://www.ucalgary.ca/fenton/2013chart).

The term "growth and/or development" as used herein (not pertaining to the invention) may refer to growth and/or development of the brain of a preterm infant and/or the cognitive function of the infant, as further defined below, and/or to growth and/or development of the body of a preterm infant and/or the infant's body composition, also as further defined below.

*Brain and Cognitive Function (not pertaining to the invention)*

Balanced growth and/or development may refer to the balanced growth and/or development of the brain of a preterm infant and/or the cognitive function of the infant. This may refer to physical development of the brain such as increased brain size, neuron proliferation and apoptosis, synapsis formation, rearrangement and/or number of synaptic connection, as well as neurotransmitter activity and also head circumference. This may also refer to any brain function or behaviour observed in the infant or later in the life of the infant, such as visual development, sensory perception, language, higher cognitive functions (such as logic) but also emotional control, habits, mood, appetite, sleep, memory, learning, and some social skills and behaviours of the infant. It may also refer to preventing or reducing the infant's risk for developing a psychiatric disease in later life (*e.g.,* an affective disorder, depression, schizophrenia).

As described herein, the recommended intake determined for phenylalanine, an essential amino acid that is associated with brain function, was found to be different to those found in commercially available preterm formulas. Furthermore, it is believed that the ratio between phenylalanine and the other large neutral amino acids tryptophan, valine, leucine, isoleucine, tyrosine and methionine and is also significantly different to the recommended ratio, wherein it has been stated that an altered ratio might have possible implications on brain development. It is therefore believed that preterm formulas that comprise the modified phenylalanine concentrations as herein defined advantageously promote, assist, or achieve balanced growth or development of brain and cognitive function in preterm infants.

*Body Growth, Development and Composition (not pertaining to the invention)*

Balanced growth and/or development may refer to the balanced growth and/or development of the body of a preterm infant and/or the infant's body composition. This may refer to the height, weight, fat distribution (*e.g.*, visceral fat versus subcutaneous fat), or other parameter of the infant, such parameters being well known to those skilled in the art.

## EXAMPLES

**Example 1: Amino acid (phenylalanine) requirements in preterm infants**

This example describes the determination of the requirements of the essential amino acid phenylalanine in preterm infants by way of the indicator amino acid oxidation (IAAO) method, and the subsequent definition of the recommended ranges of dietary requirements for this essential amino acid.

These infants (n=16) all had a gestational age of ≤37 weeks, and a weight of ≤2500 on study day. They were clinically stable and exhibited a weight gain rate of 10 g·kg$^{-1}$·d$^{-1}$ in the preceding 3 days.

## IAAO Method

In the IAAO method, each infant was fed an amino acid based formula where each essential amino acid was present in excess except the test amino acid phenylalanine. The infant was randomly assigned to receive the test amino acid in an amount ranging from deficient to excess (*i.e.,* each infant was given one specific intake level of the test amino acid for one test period). Lysine, labelled with a stable isotope, was used as the indicator amino acid for assessing phenylalanine requirement.

The IAAO method (Zello, 1993) is based on the concept that when the test amino acid intake is insufficient to meet the infant's requirements, protein synthesis will be limited and all of the amino acids will be oxidised, including the indicator amino acid. Oxidation of the indicator amino acid can be measured in expired air as $^{13}CO_2$.

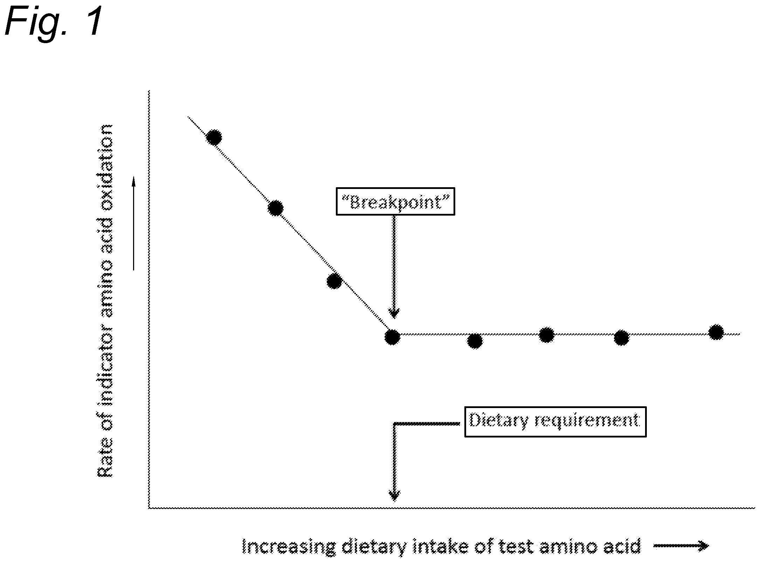

Figure 1 shows an example of the graph plotted by the IAAO method for a test amino acid. As can be seen in Figure 1, as the dietary intake of the test amino acid (shown on the *x*-axis) increases, protein synthesis increases and the oxidation rate of the indicator amino acid (shown on the *y*-axis) decreases until the requirement of the test amino acid is met (indicated as the breakpoint). Once the requirement of the test amino acid is met, a further increase in its intake will have no further influence on the oxidation rate of the indicator amino acid. Thus the breakpoint determines the dietary requirement of the test amino acid in this setting.

## IAAO Method - Protocol

24 hours before the study day, the infants consumed their test formula (including the specific intake level of the test amino acid being given for each particular infant) for adaptation, with the feeding regime conforming to that of the hospital. During the study day, the feeding regime was changed to hourly bolus feeding of the infant's test formula to ensure a metabolic steady state in feed condition. In order to quantify individual $CO_2$ production, the infants received a primed (14 μmol/kg) continuous (9 μmol/kg/h) infusion of [$^{13}$C]bicarbonate (sterile pyrogen free, 99% $^{13}$C APE; Cambridge Isotopes, Woburn, MA) for 2.5 hours (Riedijk, 2005). Directly following the bicarbonate infusion, a primed (34 μmol/kg) continuous (40 μmol/kg/h) enteral infusion of [1-$^{13}$C]lysine 2HCl (99% $^{13}$C APE; Cambridge Isotopes) was given for 4.5 hours. To minimise invasiveness the tracers were given enterally by means of a gastric tube. An excess of tyrosine (166 mg/kg/d) was used.

Breath samples were collected by means of the direct sampling method described by van der Schoor (2004). At baseline, two duplicate breath samples were obtained before tracer infusion. During the last 45 minutes of [$^{13}$C] bicarbonate infusion, and during the last hour of [1-$^{13}$C]lysine 2HCl infusion, duplicated samples were collected every ten and fifteen minutes, respectively. Samples were stored at room temperature until monthly shipment to the Netherlands for analysis at the mass spectrometry laboratory of the Erasmus Medical Center, Rotterdam. Expired $^{13}CO_2$ enrichment was measured by isotope mass spectrometry (ABCA; Europe Scientific, van Loenen Instruments, Leiden, the Netherlands), and reported in units of atom percent excess (APE).

For each participant, the estimated body $CO_2$ production was calculated, as described previously (Riedijk, 2005). The rate of fractional [1-$^{13}$C]lysine 2HCl oxidation was calculated using this equation:

$$\text{Fractional lysine oxidation (\%)} = [\text{IE}_{\text{LYS}} \times \text{i}_{\text{B}}]/[\text{i}_{\text{LYS}} \times \text{IE}_{\text{B}}] \times 100\%,$$

where $\text{IE}_{\text{LYS}}$ is the excess $^{13}$C isotopic enrichment in expired air during [1-$^{13}$C]lysine 2HCl infusion (APE), $\text{i}_{\text{B}}$ is the infusion rate of [$^{13}$C]bicarbonate ($\mu$mol/kg/h), $\text{i}_{\text{LYS}}$ is the infusion rate of [1-$^{13}$C]lysine 2HCl ($\mu$mol/kg/h), and $\text{IE}_{\text{B}}$ is the excess $^{13}$C isotopic enrichment in expired air during [$^{13}$C]bicarbonate infusion (van Goudoever, 1993).

**Phenylalanine - Requirement**

Using the method described above, the 16 infants as previously described were randomly assigned to phenylalanine intakes ranging from 5 to 177 mg/kg/d. There were 24 test intakes for the 16 preterm infants. The breakpoint for phenylalanine by the group of 16 infants (24 test intakes) was estimated to be 80 mg·kg$^{-1}$·d$^{-1}$.

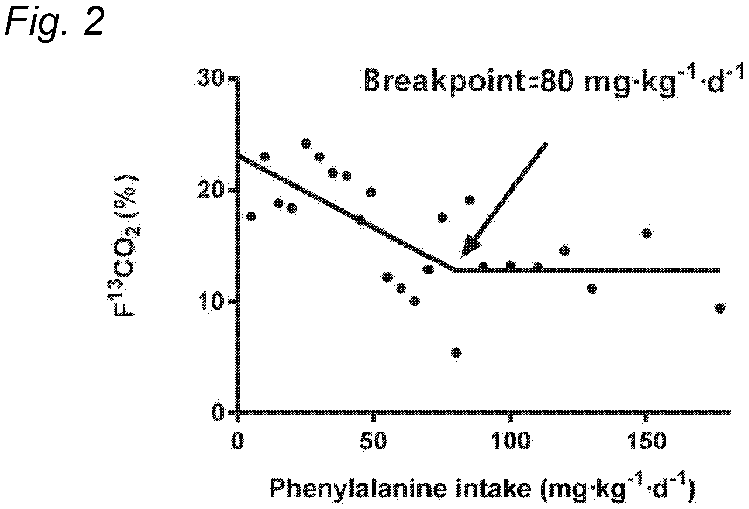

These results are also illustrated graphically in Figure 2. In Figure 2, it is the oxidation rate of [1-$^{13}$C]lysine 2HCl against dietary intake of the test amino acid (phenylalanine) that is plotted. Each point represents the result of a specific test intake of an individual infant who was fed an amino acid based formula where each essential amino acid was present in excess except phenylalanine, where phenylalanine was present in an amount ranging from deficient to excess. There were 24 test intakes for the 16 preterm infants.

**Phenylalanine - Results and calculations**

**(1) Dietary requirement / recommended intake**

The recommended dietary intake range for phenylalanine was then determined using the following strategy.

First, the dietary requirement (breakpoint) of the IAAO results was estimated using a biphasic linear regression crossover model and was taken as the primary parameter.

**The dietary requirement (breakpoint) for phenylalanine in preterm infants was estimated to be 80 mg·kg$^{-1}$·d$^{-1}$ as discussed above and also shown in Figure 2.**

To ensure that nearly all individuals meet the amino acid requirements, a safe level of intake was defined. The safe level of protein intake proposed by the WHO (WHO/FAO/UNU, 2007) was used, defining the safe level of protein intake as 125% of the average protein requirement. In this instance, the safe level of amino acid intake was therefore calculated to be >125% of the primary parameter (breakpoint value) obtained as described above, to reach the population safe requirement value.

An assumption was made that this primary parameter (breakpoint value) is higher than the requirement of protein bound amino acids based on the study by Metges (Metges, 2000) which showed > 20% higher first pass oxidation rate when free amino acids are ingested compared to protein bound amino acids. Therefore 20% of the estimated requirement was subtracted from the population safe requirement value, which is believed to be conservative (*i.e.,* an overestimate rather than underestimate).

The net effect of the above two calculations is therefore that the breakpoint equals the recommended intake (measured in mg·kg$^{-1}$·d$^{-1}$).

**Therefore the recommended intake of phenylalanine in preterm infants was established as 80 mg·kg$^{-1}$·d$^{-1}$.**

**(2) Recommended intake levels in other units (*e.g.,* for preterm formulas)**

The recommended intake measured in 80 mg·kg$^{-1}$·d$^{-1}$ was then converted to units of g (phenylalanine) / 100 g protein based on the ESPGHAN recommendations which provide a guideline to provide 3.5 to 4.0 g protein per kg body weight per day for preterm infants weighing 1.0-1.8 kg. Thus, the recommended intake expressed in mg·kg$^{-1}$·d$^{-1}$ was divided separately by 3.5 g·kg$^{-1}$·d$^{-1}$ and by 4.0 g·kg$^{-1}$·d$^{-1}$ to reach a recommended dietary intake expressed in g (phenylalanine) / 100 g (protein).

**Therefore the recommended intake of phenylalanine in preterm infants was determined to be 2.0-2.29 g phenylalanine / 100 g protein.**

The recommended intake was also converted into units of g (protein) / 100 kcal (energy intake). This conversion also used the ESPGHAN guideline to provide 3.5 to 4.0 g protein per kg body weight per day for preterm infants weighing 1.0-1.8 kg, in combination with the recommended protein intake per day for preterm infants of 3.3 g (total protein) / 100 kcal. The calculations are indicated in Table 1 below, where it can be seen that the recommended intake level for phenylalanine in preterms in mg (phenylalanine) / 100 kcal was determined by multiplying the recommended intake expressed in mg (phenylalanine) / g (protein) by the energy intake levels of 3.2-3.6 g (protein) / 100 kcal.

Table 1: Calculations to determine recommended intake for phenylalanine in preterm infants expressed by way of energy intake

| Breakpoint (mg/kg/d) | Protein level (g/kg/d) | Phe levels (mg/g protein) | Energy (g protein/ 100 kcal) | Phe level (mg/ 100 kcal) | |
|---|---|---|---|---|---|
| 80 | 3.5 | 22.9 | 3.2 | 22.9 x 3.2 | = 73.14 |
| 80 | 3.5 | 22.9 | 3.6 | 22.9 x 3.6 | = 82.29 |
| 80 | 4.0 | 20.0 | 3.2 | 20.0 x 3.2 | = 64.00 |
| 80 | 4.0 | 20.0 | 3.6 | 20.0 x 3.6 | = 72.00 |

Therefore the recommended intake of phenylalanine in preterm infants was also determined to be 64.00-82.29 mg phenylalanine / 100 kcal.

**(3) Human milk fortifier levels**

For consumption of fortified human milk, the same intake recommendations apply as those of preterm formulas (as calculated above). The recommended intake levels that are calculated above were also used in the determination of optimal phenylalanine levels in a human milk fortifier, taking into account the phenylalanine concentration in human milk.

Data on essential amino acid concentrations in preterm milk is sparse. Zhang and colleagues presented meta-analysis data for composition of transitional preterm milk (~day 6-20) and compared the composition of this milk to transitional term milk (Zhang, 2013). However, from the same study it is clear that transitional (term) milk is an intermediate state in the lactation phase and varies compared to mature (term) milk (> day 20). In this publication, concentrations of phenylalanine were 79.1 and 63.8 mg/100 ml milk in preterm and term milk, respectively. However, the term milk concentrations at 2 months expression were much lower (46.0 mg/100 ml). Since preterm infants often receive donor milk, which in turn is most often mature term milk, the inventors used the term milk concentrations at 2 months lactation in determining the degree of fortification required to meet protein intake requirements (3.5-4.0 g/kg/d) without exceeding preterm requirements for phenylalanine (80 mg/kg/d).

Thus the calculations to determine the level of phenylalanine needed in a human milk fortifier (to supplement human milk) was determined based on human milk having the characteristics shown in Table 2.

Table 2: Characteristics of human milk (after 2 months lactation)

| Human milk (2 months) | |
|---|---|
| Phe (mg/100 ml) | 46.0 |
| Protein level (Nx6.25) | 1.31 |
| Caloric content (kcal/100 ml) | 65 |
| Protein content (g/100 kcal) | 2.02 |
| Phe (mg/g protein) | 35.11 |
| Phe (mg/100 kcal) | 70.77 |

First, the **protein gap** that needs to be filled with human milk fortifier (in order to meet protein requirements of preterm infants) was determined to be **1.185-1.585 g protein / 100 kcal HMF** based on a target protein of between 3.2-3.6 g (protein) / 100 kcal minus the protein content provided by the human milk as shown in Table 2 to be 2.02 g (protein) / 100 kcal.

Second, the **phenylalanine gap** that needs to be filled with human milk fortifier (in order to meet protein requirements of preterm infants) was determined by subtracting the phenylalanine content in the human milk (70.77 mg/100 kcal as shown in Table 2) from the recommended intake of phenylalanine in preterm infants of 64.00-82.29 mg phenylalanine / 100 kcal. This gives a range of **0-11.52 mg phenylalanine / 100 kcal in a human milk fortifier.** Based on total protein in the HMF of 1.185-1.585 g protein / 100 kcal HMF (as determined above), this also translates into **0-9.72 mg phenylalanine / g protein.**

These results provide a recommendation for a human milk fortifier to provide no additional phenylalanine beyond the phenylalanine level in human milk, or to provide a very low level of phenylalanine (up to approximately 11.52 mg / 100 kcal).

**Discussion**

Recommendations directed at intake of specific essential amino acids in preterm infants currently do not exist. In the experiments described herein, the requirements for phenylalanine in preterm infants was found to be lower than the current intake levels of preterm infants that are either fed existing preterm formulas or human milk fortified with a human milk fortifier. For example, in cow's milk-based preterm formulas, phenylalanine levels have been reported to vary between 3.54-4.53 g (phenylalanine) / 100 g protein (Picaud, 2001; Moro, 1999; Darling, 1999; Szajewska, 2001) or 91.64-118.25 mg (phenylalanine) / 100 kcal (Picaud, 2001; Moro, 1999; Darling, 1999; Szajewska, 2001).

Preterm infants require high levels of protein (considerably higher than term infants), and therefore these results suggesting a lower level of one of the essential amino acids were unexpected. Without wishing to be bound by theory, the inventors postulate that these results may indicate that it is the relative concentration of phenylalanine to the concentrations of the other amino acids (and not the total protein levels) that need to be changed in existing preterm formulations, particularly preterm formulas and human milk fortifiers.

For instance, phenylalanine and other large neutral amino acids compete for transport across the blood-brain barrier via the L-type amino acid carrier, as well as at the gut-blood barrier (Pietz, 1999). Elevated plasma phenylalanine concentrations thus impair brain uptake of other large neutral amino acids, and is seen most acutely in patients suffering from phenylketonutria (PKU). Direct effects of elevated phenylalanine concentrations and depleted large neutral amino acid concentrations in the brain are probably major causes for disturbed brain development and function in PKU. One of the therapeutic strategies in PKU is supplementing large neutral amino acids to the patients, based on the rationale that this will favourably increase the transport of large neutral amino acids at the blood-brain barrier and also at the gut-blood barrier, resulting in lower phenylalanine concentrations in the brain and plasma. The insights from the present invention, namely that preterm infants require less phenylalanine than is currently provided in preterm formulations, particularly preterm formulas and human milk fortifiers, and thus the proposition that phenylalanine concentrations should be reduced relative to other amino acids in preterm formulations, mean that the large neutral amino acids involved in protein accretion will have a better bioavailability due to less transporter competition. As a result, the lower ratio of phenylalanine to other amino acids, especially the large neutral amino acids, may promote, assist or achieve balanced growth or development of a preterm infant, and in particular the growth or development of the brain of a preterm infant.

## REFERENCES

1. Agostoni, C., Buonocore, G., Carnielli, V.P., De Curtis, M., Darmaun, D., Decsi, T., Domellof, M., Embleton, N.D., Fusch, C., Genzel-Boroviczeny, O., Goulet, O., Kalhan, S.C., Kolacek, S., Koletzko, B., Lapillonne, A., Mihatsch, W., Moreno, L., Neu, J., Poindexter, B., Puntis, J., Putet, G., Rigo, J., Riskin, A., Salle, B., Sauer, P., Shamir, R., Szajewska, H., Thureen, P., Turck, D., Van Goudoever, J.B. and Ziegler, E.E. "Enteral Nutrient Supply for Preterm Infants: Commentary from the European Society of Paediatric Gastroenterology, Hepatology and Nutrition Committee on Nutrition." J. Pediatr. Gastroenterol. Nutr., 2010: 50(1); 85-91.

2. Darling, P.B. et al., "Threonine kinetics in preterm infants fed their mothers' milk or formula with various ratios of whey to casein" Am J Clin Nutr 1999 Jan: 69(1); 105-114.

3. Klein, C.J. "Nutrient Requirements for Preterm Infant Formulas." J. Nutr. 2002: 132(6 Suppl 1); 1395S-1577S.

4. Metges, C.C. et al., "Kinetics of L-[1-(13C)]leucine when ingested with free amino acids, unlabeled or intrinsically labeled casein" Am J Physiol Endocrinol Metab 2000: 278(6); E1000-9.

5. Moro G., Minoli I., Boehm G., Georgi G., Jelinek J., Sawatzki G., "Postprandial plasma amino acids in preterm infants: influence of the protein source." Acta Paediatr 1999: 88; 885-889.

6. Picaud J-C., Rigo J., Normand S., Lapillonne A., Reygrobellet B., Claris O., Salle B. L., "Nutritional Efficacy of Preterm Formula With a Partially Hydrolyzed Protein Source: A Randomized Pilot Study" Journal of Pediatric Gastroenterology and Nutrition 2001 May: 32; 555-561.

7. Riedijk M.A., Voortman G., van Goudoever J.B., "Use of [13C]bicarbonate for metabolic studies in preterm infants: intragastric versus intravenous administration." Pediatr Res 2005: 58; 861-4.

8. Szajewska H., Albrecht P., Stoinska B., Prochowska A., Gawecka A., Laskowska-Klita T., "Extensive and Partial Protein Hydrolysate Preterm Formulas: The Effect on Growth Rate, Protein Metabolism Indices, and Plasma Amino Acid Concentrations" Journal of Pediatric Gastroenterology and Nutrition 2001 March: 32; 303-309.

9. van der Schoor, S.R. et al., "Validation of the direct nasopharyngeal sampling method for collection of expired air in preterm neonates." Pediatr Res 2004: 55(1); 50-54.

10. van Goudoever J.B., Sulkers E.J., Chapman T.E., et al. "Glucose kinetics and glucoregulatory hormone levels in ventilated preterm infants on the first day of life." Pediatr Res 1993: 33; 583-9.

11. WHO MULTICENTRE GROWTH REFERENCE STUDY GROUP "WHO Child Growth Standards based on length/height, weight and age" Acta Paediatrica 2006: Suppl 450; 76-85.

12. WHO/FAO/UNU, Protein and amino acid requirements in human nutrition. WHO Technical Report Series, 2007(935): p. 1-265.

13. Zello, G.A. et al., "Dietary lysine requirement of young adult males determined by oxidation of 1-[1-13C]phenylalanine" Am J Physiol Endocrinol Metab 1993: 264; E677-E685.

14. Zhang Z., Adelman A.S., Rai D., Boettcher J., Lonnerdal B., "Amino Acid Profiles in Term and Preterm Human Milk through Lactation: A Systematic Review" Nutrients 2013 Nov 26; 5(23):4800-21.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

## Claims

1. An enteral preterm infant formula comprising a proteinaceous composition, wherein the preterm infant formula:

   a) comprises 2.0-2.29 g phenylalanine per 100 g protein;
   b) comprises 64.00-82.29 mg phenylalanine per 100 kcal; and/or
   c) provides, or is formulated to provide phenylalanine in an amount of 80-96 mg / kg body weight per day.

2. The preterm infant formula according to claim 1, comprising between 35 and 50 en% lipid, between 7.5 and 18 en% protein and between 35 and 80 en% carbohydrate, wherein en% is short for energy percentage and represents the relative amount each constituent contributes to the total caloric value of the preparation.

3. The preterm infant formula according to claim 1, comprising 3.2 - 3.6 g protein per 100 kcal.

4. Preterm infant formula for use in promoting, assisting or achieving balanced growth or development in a preterm infant and/or for use in preventing or reducing the risk of unbalanced growth or development in a preterm infant, comprising enterally administering a preterm infant formula according to any one of claims 1-3 to said preterm infant.

## Patentansprüche

1. Enteraler Muttermilchersatz für frühgeborene Kinder, umfassend eine proteinhaltige Zusammensetzung, wobei der Muttermilchersatz für frühgeborene Kinder:

   a) 2,0 - 2,29 g Phenylalanin pro 100 g Protein umfasst;
   b) 64,00 - 82,29 mg Phenylalanin pro 100 kcal umfasst; und/oder
   c) Phenylalanin in einer Menge von 80 - 96 mg/kg Körpergewicht pro Tag liefert oder formuliert ist, um Phenylalanin in einer Menge von 80 - 96 mg/kg Körpergewicht pro Tag zu liefern.

2. Muttermilchersatz für frühgeborene Kinder nach Anspruch 1, umfassend zwischen 35 und 50 en% Lipid, zwischen 7,5 und 18 en% Protein und zwischen 35 und 80 en% Kohlenhydrat, wobei en% eine Kurzform für den Energieprozentsatz ist und die relative Menge darstellt, die jeder Bestandteil zum Gesamtkaloriengehalt der Zubereitung beiträgt.

3. Muttermilchersatz für frühgeborene Kinder nach Anspruch 1, umfassend 3,2 - 3,6 g Protein pro 100 kcal.

4. Muttermilchersatz für frühgeborene Kinder zur Verwendung bei der Förderung, Unterstützung oder Erzielung eines ausgewogenen Wachstums oder einer ausgewogenen Entwicklung bei einem frühgeborenen Kind und/oder zur Verwendung bei der Prävention oder Verringerung des Risikos von unausgewogenem Wachstums oder unausgewogener Entwicklung bei einem frühgeborenen Kind, umfassend die enterale Verabreichung eines Muttermilchersatzes für frühgeborene Kinder nach irgendeinem der Ansprüche 1 - 3 an das frühgeborene Kind.

**Revendications**

1.  Formulation entérale pour nourrissons prématurés, comprenant une composition protéique, dans laquelle la formulation pour nourrissons prématurés :

    a) comprend entre 2,0 et 2,29 g de phénylalanine pour 100 g de protéines ;
    b) comprend entre 64,00 et 82,29 mg de phénylalanine pour 100 kcal ; et/ou
    c) fournit, ou est formulée pour fournir, une quantité de phénylalanine comprise entre 80 et 96 mg/kg de masse corporelle par jour.

2.  Formulation pour nourrissons prématurés selon la revendication 1, comprenant entre 35 et 50 en% de lipides, entre 7,5 et 18 en% de protéines et entre 35 et 80 en% de glucides, dans laquelle en% désigne de manière abrégée le pourcentage en énergie et représente la quantité relative de chaque constituant contribuant à la valeur calorique totale de la préparation.

3.  Formulation pour nourrissons prématurés selon la revendication 1, comprenant entre 3,2 et 3,6 g de protéines pour 100 kcal.

4.  Formulation pour nourrissons prématurés destinée à être utilisée pour favoriser, assister ou assurer la croissance ou le développement équilibré d'un nourrisson prématuré et/ou destinée à être utilisée pour empêcher ou réduire le risque de croissance ou de développement déséquilibré d'un nourrisson prématuré, comprenant l'administration entérale d'une formulation pour nourrissons prématurés selon l'une quelconque des revendications 1 à 3 audit nourrisson prématuré.

*Fig. 1*

*Fig. 2*

Breakpoint=80 mg·kg⁻¹·d⁻¹

F¹³CO₂ (%)

Phenylalanine intake (mg·kg⁻¹·d⁻¹)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001041581 A

### Non-patent literature cited in the description

- **BALLHAUSEN et al.** *Pediatric Reports,* 2011, vol. 3, 103-107
- **AGOSTONI, C. ; BUONOCORE, G. ; CARNIELLI, V.P. ; DE CURTIS, M. ; DARMAUN, D. ; DECSI, T. ; DOMELLOF, M. ; EMBLETON, N.D. ; FUSCH, C. ; GENZEL-BOROVICZENY, O.** Enteral Nutrient Supply for Preterm Infants: Commentary from the European Society of Paediatric Gastroenterology, Hepatology and Nutrition Committee on Nutrition. *J. Pediatr. Gastroenterol. Nutr.,* 2010, vol. 50 (1), 85-91
- **DARLING, P.B. et al.** Threonine kinetics in preterm infants fed their mothers' milk or formula with various ratios of whey to casein. *Am J Clin Nutr,* January 1999, vol. 69 (1), 105-114
- **KLEIN, C.J.** Nutrient Requirements for Preterm Infant Formulas. *J. Nutr.,* 2002, vol. 132 (1), 1395S-1577S
- **METGES, C.C. et al.** Kinetics of L-[1-(13)C]leucine when ingested with free amino acids, unlabeled or intrinsically labeled casein. *Am J Physiol Endocrinol Metab,* 2000, vol. 278 (6), E1000-9
- **MORO G. ; MINOLI I. ; BOEHM G. ; GEORGI G. ; JELINEK J. ; SAWATZKI G.** Postprandial plasma amino acids in preterm infants: influence of the protein source. *Acta Paediatr,* 1999, vol. 88, 885-889
- **PICAUD J-C. ; RIGO J. ; NORMAND S. ; LAPILLONNE A. ; REYGROBELLET B. ; CLARIS O. ; SALLE B. L.** Nutritional Efficacy of Preterm Formula With a Partially Hydrolyzed Protein Source: A Randomized Pilot Study. *Journal of Pediatric Gastroenterology and Nutrition,* May 2001, vol. 32, 555-561
- **RIEDIJK M.A. ; VOORTMAN G. ; VAN GOUDOEVER J.B.** Use of [13C]bicarbonate for metabolic studies in preterm infants: intragastric versus intravenous administration. *Pediatr Res,* 2005, vol. 58, 861-4
- **SZAJEWSKA H. ; ALBRECHT P. ; STOINSKA B. ; PROCHOWSKA A. ; GAWECKA A. ; LASKOWSKA-KLITA T.** Extensive and Partial Protein Hydrolysate Preterm Formulas: The Effect on Growth Rate, Protein Metabolism Indices, and Plasma Amino Acid Concentrations. *Journal of Pediatric Gastroenterology and Nutrition,* March 2001, vol. 32, 303-309
- **VAN DER SCHOOR, S.R. et al.** Validation of the direct nasopharyngeal sampling method for collection of expired air in preterm neonates. *Pediatr Res,* 2004, vol. 55 (1), 50-54
- **VAN GOUDOEVER J.B. ; SULKERS E.J. ; CHAPMAN T.E. et al.** Glucose kinetics and glucoregulatory hormone levels in ventilated preterm infants on the first day of life. *Pediatr Res,* 1993, vol. 33, 583-9
- WHO Child Growth Standards based on length/height, weight and age. Acta Paediatrica. WHO MULTICENTRE GROWTH REFERENCE STUDY GROUP, 2006, 76-85
- WHO/FAO/UNU, Protein and amino acid requirements in human nutrition. *WHO Technical Report Series,* 2007, vol. 935, 1-265
- **ZELLO, G.A. et al.** Dietary lysine requirement of young adult males determined by oxidation of 1-[1-13C]phenylalanine. *Am J Physiol Endocrinol Metab,* 1993, vol. 264, E677-E685
- **ZHANG Z. ; ADELMAN A.S. ; RAI D. ; BOETTCHER J. ; LONNERDAL B.** Amino Acid Profiles in Term and Preterm Human Milk through Lactation: A Systematic Review. *Nutrients,* 26 November 2013, vol. 5 (23), 4800-21